# EUROPEAN PATENT SPECIFICATION

(11) **EP 2 462 908 B1**
(45) Date of publication and mention of the grant of the patent: **25.09.2013**
(21) Application number: 11001481.8
(22) Date of filing: 22.02.2011
(51) Int. Cl.: A61F 13/00, A61L 15/00, A61M 1/00

(54) **Medical dressing and negative pressure wound therapy apparatus using the same**
Medizinische Wundauflage und Unterdruckwundbehandlungsvorrichtung damit
Pansement médical et appareil de thérapie de blessures de pression négative l'utilisant

(30) Priority: 10.12.2010 TW 99143176
(43) Date of publication of application: 13.06.2012
(73) Proprietor: INDUSTRIAL TECHNOLOGY RESEARCH INSTITUTE, Chutung Chen, Hsinchu Hsien, Taiwan 310 (TW)
(72) Inventor: Chen, Jui-Hsiang, Hsinchu City 300 (TW); Chen, Yu-Hua, North Dist. Hsinchu City 300 (TW); Shih, Ting-Yu, Wenshan Dist. Taipei City 116 (TW); Hu, Wei, Zhudong Township Hsinchu County 310 (TW); Yang, Jean-Dean, Dayuan Township Taoyuan County 337 (TW); Lee, Chun-Min, Xiangshan Dist. Hsinchu City 300 (TW)
(74) Representative: Manitz, Finsterwald & Partner GbR

(56) References cited:
- WO-A1-2007/046806
- WO-A1-2009/146441
- WO-A1-2010/011434
- WO-A2-03/086232
- GB-A- 2 415 908

## Description

### BACKGROUND OF THE INVENTION

### 1. Field of the Invention

The present invention relates to medical dressings, and more particularly, to a medical dressing having polymeric fibers with repeat units containing urethane group..

### 2. Description of Related Art

A number of medical resources are used to take care of chronic wounds which result from diabetes, a neurological disorder, a pressure sore, a bedsore, a vascular disorder and etc. Hence, it is important to develop treatment for chronic wounds. Generally, medical dressings for chronic wounds are classified into conventional dressings such as bandages, gauze, band-aid and so on; wet dressings such as hydrogel, hydrocolloid and foam; and negative pressure wound therapy.

In 1874, the first commercial medical dressing was germfree absorbent cotton manufactured from a Germany company and used for covering wounds. This absorbent cotton is produced in low cost and easy to use, such that it is in use to date. However, the conventional dressings such as gauzes or cottons are capable of protecting wounds but fail to effectively separate wounds from external environment, such that the wounds may be repeatedly infected.

In recent years, active and non-invasive therapy was developed to treat wounds which are hard to heal such as bedsores, pressure sores and diabetes ulcers. The negative pressure wound therapy (NPWT) was thus developed.

Negative pressure wound therapy (NPWT) is a therapeutic technique to place a dressing between wounds and an air pump and to create pressure (about 50 to 120 mm-Hg), such that wound fluids can be removed, edema is reduced, and tissue proliferation and local blood circulation are enhanced by mechanical strength.

Currently, the negative pressure wound dressings are polyurethane foams or gauzes. However, the foam dressing is not soft enough and thus makes patient uncomfortable. Furthermore, the foam dressing is not easy to be cut into desired shape, and is not easy to be completely removed without damaging wounds and causing pain. In addition, the foam dressing lacks a steric structure support, and thus when the foam dressings are compressed by pressure, the foam dressings fail to effectively form channels for wound fluid. Moreover, sterile gauzes cannot maintain a moist environment for wounds and new tissues easily grow into fibers of gauzes, thereby resulting in damages to wounds while changing dressings.

Hence, there is a need to develop a dressing for negative pressure wound therapy especially with improvements in pressure distribution on wounds, removal of wound fluid, prevention of wound damage and operation.

### SUMMARY OF THE INVENTION

The present invention provides a medical dressing, including a first and a second woven layers respectively comprising a fiber of polyurethane or a yarn of polyurethane; and a plurality of linear support portions woven between the first and second woven layer wherein the first and the second woven layer respectively have a surface with a plurality of openings, with the average area of the openings being in a range from 1 × 10⁻⁴ to 1 × 10² mm², wherein the medical dressing has an overall density in a range from 0.01 to 0.99 g/cm³, and wherein the fiber or yarn has a hardness in a range from Shore 50A to Shore 80D.

The material including polyurethane is processed to form fibers or yarns, and then woven into the first and the second woven layers. The fibers are single fibers, multiple fibers or a combination thereof. The yarns are single-strand yarns, multiple-strand yarns or a combination thereof. The first and the second woven layers respectively have a surface with a plurality of openings, and the average area of the openings is in a range of from 1 × 10⁻⁴ to 1 × 10² mm².

The medical dressing of the present invention can be used for generally protecting wounds. In order to control the flow rate of wound fluid and moisture of the wound bed, the first and the second woven layers of the medical dressing may be adjusted to have different areas of openings. When the openings of the woven layer contacting wounds are larger (the average area of the openings may be from 1 × 10⁻² to 1 × 10² mm²) and the openings of the woven layer contacting air are smaller (the average area of the openings may be from 1 × 10⁻⁴ to 1 mm²), the asymmetric structure of openings well control removal of wound fluid and moisture vapor transmission rate (MVTR). In the present invention, MVTR is in a range of from 50 to 50,000 g/m²/24hr, and preferably in a range of from 100 to 20,000 g/m²/24hr. Further, the hydrophilicity/hydrophobicity of the first and the second woven layers is controlled for the removal of wound fluid and wound moisture. Specifically, the hydrophobic material may include a polymer with repeat units of urethane; and the hydrophilic material may include a polymer with repeat units of urethane and hydrophilic segments.

In the present invention, the sizes of the openings and hydrophilicity/hydrophobicity of the first and the second woven layers, hydrophilicity/hydrophobicity of the linear support portions and the space density of the linear support portions may be adjusted, so as to immediately absorb a lot of wound fluid. Specifically, the fibers or yarns are made of the hydrophilic/hydrophobic material including repeat units of urethane and repeat units of urethane with hydrophilic segments. Therefore, the hydrophilic fibers or yarns are capable of absorbing water or wound fluid, and the absorption capability is 5% to 1000%, and preferably 10% to 500%, of the unit volume of the dressing.

In the present invention, the sizes of openings and hydrophilicity/hydrophobicity of the first and the second woven layers, hydrophilicity/hydrophobicity of the linear support portions and the space density of the linear support portions may be adjusted, so as to be applicable for negative pressure wound therapy (NPWT). Specifically, the woven layer contacting wounds may have smaller openings, so as to facilitate flow of wound fluid and to prevent new tissues from growing into the interior of the dressing. Thus, while changing dressings, the newly grown tissues would not be damaged, thereby avoiding patients from suffering pain. Moreover, the average area of openings of the first woven layer (contacting wounds) may be less than 1 mm², and preferably from 1×10⁻⁴ to 1 mm²; and the average area of openings of the second woven layer may be more than 1 × 10⁻² mm², and more preferably from 1 × 10⁻² to 1 × 10² mm². Alternatively, the first and the second woven layers may have the same average area of openings, i.e. 1 × 10⁻⁴ to 1 × 10² mm².

When the medical dressing of the present invention is used for negative pressure wound therapy, the support portions between the first and the second woven layers have a designed pore density so as to have desired support strength for capillary channel for fluid flow. Specifically, when the medical dressing of the present invention is used for negative pressure wound therapy, the blood mimicking fluid (Lot#CGB3120, Model 046, Computerized Imaging Reference Systems, Inc.) has flow rate more than 50 mg/sec, and preferably more than 70 mg/sec, at 600 mm-Hg. While the dressing is applied on wounds for negative pressure wound therapy, the difference between the pressure distributions may be less than 10 mm-Hg, and preferably less than 5 mm-Hg.

In one embodiment of the present invention, the polyurethane includes aliphatic polyurethane, aromatic polyurethane, aliphatic polyurethane having a hydrophilic segment, and aromatic polyurethane having a hydrophilic segment or a combination thereof. The hydrophilic segment is positioned on a main chain or is a side chain bound to the aliphatic polyurethane or the aromatic polyurethane. For example, the hydrophilic segment is bound to the aliphatic polyurethane or the aromatic polyurethane. Further, the hydrophilic segment includes polyethylene glycol (PEG), polyethylene oxide (PEO), polyvinylpyrrolidone (PVP), polyvinyl alcohol (PVA), polyacrylic acid (PAA), and polymethacrylic acid (PMA) or a combination thereof.

In one embodiment, the hydrophilic segment has a structure of formula (I) or formula (II): 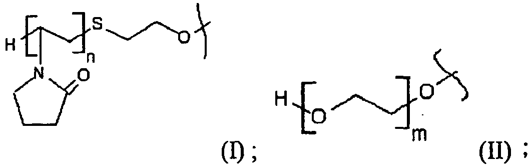 wherein n is an integer of from 1 to 20,000; m is an integer of from 1 to 50,000; and the hydrophilic segment of formula (I) or formula (II) is the side chain bound to the polymer.

In one embodiment, the polymer includes repeat units of urethane and is formed from a reaction of 4,4'-diphenylmethane diisocyanate and a hydrophilic segment having a glycol group and a structure of formula (III) or formula (IV) 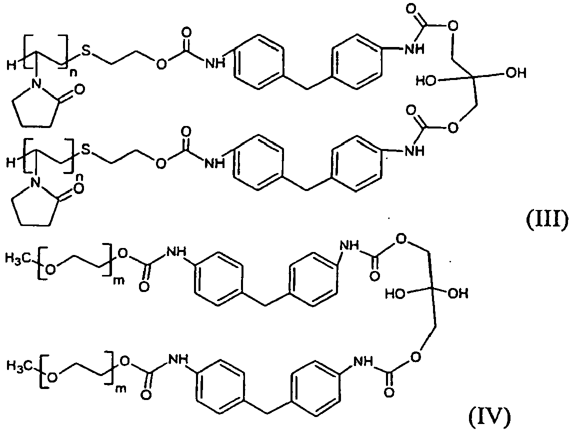 , wherein n is an integer of from 1 to 20,000; and m is an integer of from 1 to 50,000.

In one embodiment, the medical dressing includes an antimicrobial component selected from the group consisting of silver, iodine, zinc, copper and an antibiotic. The antimicrobial component is disposed on a surface of the first woven layer, the second woven layer or the plurality of linear support portions. The antimicrobial component is coupled to the surface of the first woven layer, the second woven layer or the plurality of linear support portions via a coupling agent.

In addition, when the medical dressing is disposed in an environment with local negative pressure higher than 600 mm-Hg, the average rate of fluid flowing through the medical dressing is more than 50 mg/sec. Preferably, when the local negative pressure is 635 mm-Hg, the fluid flow rate of the medical dressing is more than 110 mg/sec.

The present invention further provides a negative pressure wound therapy apparatus, including a medical dressing having a first and a second woven layers respectively comprising a fiber of polyurethane or a yarn of polyurethane; and a plurality of linear support portions woven between the first and second woven layers; a sealing component attached on the medical dressing; a vacuum unit; and a communicating pipe having two ends respectively connected to the sealing component and the vacuum unit, wherein the medical dressing is filled or applied on wounds, and the sealing component is used for establishing a local negative pressure environment for wounds.

The medical dressing of the present invention has a structure with spaces and pores to produce capillary effects for absorbing wound fluid. Further, the medical dressing of the present invention is soft and can fit wounds due to fibers or yarns made of polyurethane. Moreover, the medical dressing of the present invention has the polymer material including repeat units of urethane and hydrophilic segments, so as to avoid adhesion between the dressing and the wound bed.

### BRIEFE DESCRIPTION OF THE DRAWINGS

FIG. 1 is a schematic view showing the medical dressing of the present invention;
   and
FIG. 2 is a schematic view showing the medical dressing used in the negative pressure therapy apparatus according to the present invention.

### DETAILED DESCRIPTION OF THE PREFERRED EMBODIMENTS

The detailed description of the present invention is illustrated by the following specific examples. Persons skilled in the art can conceive the other advantages and effects of the present invention based on the disclosure contained in the specification of the present invention.

The fiber processing in the present invention is well-known technology in the field, and thus the related detailed description is omitted herein.

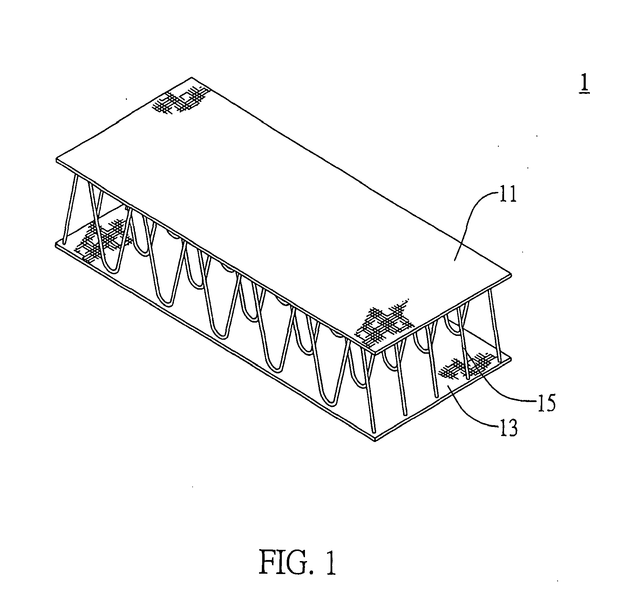

As shown in FIG. 1, the medical dressing 1 of the present invention includes a first woven layer 11 having fibers or yarns made of polyurethane; a second woven layer 13 having fibers or yarns made of polyurethane; and a plurality of linear support portions 15 woven between the first woven layer 11 and the second woven layer 13.

The material including polyurethane is processed to form fibers or yarns, and then woven into the first and the second woven layers. The fibers are single fibers, multiple fibers or a combination thereof. The yarns are single-strand yarns, multiple-strand yarns or a combination thereof. The fiber or yarn is in a range of from 0.01 denier to 1000 denier, and preferably in a range of from 0.1 denier to 500 denier.

In the present invention, the first and the second woven layers of the medical dressing have a weaving structure, a knitting structure, a circular knitting structure or a warp knitting structure, and respectively have a thickness in a range of from 0.01 to 100 mm, and preferably in a range of from 0.1 to 10 mm. Similarly, the plurality of linear support portions may be woven in the way as the first and the second woven layers.

The first and the second woven layers respectively have a surface with a plurality of openings, and the average area of the openings is in a range of from 1 × 10⁻⁴ to 1 × 10² mm², and preferably in a range of from 1 × 10⁻² to 25 mm². In the medical dressing of the present invention, there may be a plurality of openings among the linear support portions, and the openings may have the same or different areas. Thus, the medical dressing of the present invention has continuous and communicating channels to control the flow rate of the wound fluid. Further, the overall density of the medical dressing is in a range of from 0.01 to 0.99 g/cm³, and preferably in a range of from 0.1 to 0.99 g/cm³. The absorption capability of the medical dressing of the present invention is 5% to 1000% of the unit volume, and preferably 10% to 500% of the unit volume.

When the medical dressing of the present invention is used for negative pressure wound therapy, the local pressure of the wound bed is lower than atmospheric pressure so as to remove wound fluid, wherein the local pressure of the wound bed is in a range of from 759 to 500 mm-Hg. When the medical dressing of the present invention is used for negative pressure wound therapy, the difference of pressure distributions is less than 10 mm-Hg, and preferably less than 5 mm-Hg.

In order to form a soft medical dressing with better fit to the wounds and better biocompatibility, the hardness of the fiber or yarn is in a range of from Shore 50A to Shore 80D and preferably in a range of from Shore 70A to Shore 80D. The fineness of the fiber or yarn is in a range of from 0.01 denier to 1000 denier, and preferably in a range of from 0.1 denier to 500 denier.

In the present invention, the polyurethane includes aliphatic polyurethane, aromatic polyurethane, aliphatic polyurethane having a hydrophilic segment, and aromatic polyurethane having a hydrophilic segment or a combination thereof. In addition, the fiber or yarn of the medical dressing may further include thermoplastic polyester material, bio-absorbable polymeric material, antimicrobial material, material for prevent adhesion between tissues and cells, hemostatic material, material for enhancing blood circulation, and material for absorbing odor. In the preparation of woven layers, one or more of the above materials may be processed to form fibers, which are further woven as woven layers.

The polyurethane of the present invention may be aliphatic polyurethane, aromatic polyurethane, aliphatic polyurethane having a hydrophilic segment or aromatic polyurethane having a hydrophilic segment. The hydrophilic segment is positioned on a main chain or is a side chain bound to the aliphatic polyurethane or the aromatic polyurethane. The hydrophilic segment is bound to the aliphatic polyurethane or the aromatic polyurethane via an ester bond or a urethane bond.

The hydrophilic segment includes polyethylene glycol (PEG), polyethylene oxide (PEO), polyvinylpyrrolidone (PVP), polyvinyl alcohol (PVA), polyacrylic acid (PAA), and polymethacrylic acid (PMA) or a combination thereof.

In each main chain of aliphatic polyurethane or aromatic polyurethane, the repeat unit of the hydrophilic segment is more than 1, and the molecular weight of the hydrophilic segment is less than 500,000. The molecular weight of the hydrophilic segment is in a range of from 100 to 500,000, preferably less than 200,000, and more preferably less than 100,000.

The bio-absorbable polymeric material may be polycaprolactone (PCL), polylactic acid (PLA), polyglycolic acid (PGA), polylactic-co-glycolic acid copolymer (PLGA), polycaprolactone-polylactic acid copolymer (PCL-PLA), polycaprolactone-polyethylene glycol copolymer (PCL-PEG) or a combination thereof.

The antimicrobial material may be silver, iodine, zinc, copper or an antibiotic added in the surfaces of the first woven layer, the second woven layer and the plurality support portions. In order to provide the antimicrobial material on the surface, the surfaces of the first woven layer, the second woven layer and the plurality of linear support portions are immersed, sprayed or sputtered with the antimicrobial material. In order to provide the antimicrobial material on the whole medical dressing, silver, iodine, zinc, copper or an antibiotic may be added into materials for forming fibers.

Preferably, the surface is treated by immersion, wherein the antimicrobial component is added in one or more organic solvents, and then is coupled to the surface of fibers due to dissolution.

In addition, the antimicrobial component may be coupled to the fiber surface via a coupling agent, which may be the common polyurethane, aqueous polyurethane, hydrophilic polyurethane, amphiphilic polymer or a combination thereof.

The treatment of the fiber surface further includes drying, wherein the temperature for drying is in a range of from 10 to 200°C, and preferably in a range of from 25 to 120°C.

The material for avoiding adhesion between tissues and cells may be the polymer including repeat units of urethane and side chains having hydrophilic segments, and also may be the polymer including repeat units of urethane and the main chain having polyglycol, polyvinyl pyrrolidone, polyvinyl alcohol, polyacrylic acid, polymethacrytic acid or a copolymer thereof.

The hemostatic material may be oxidized cellulose (OC), oxidized regenerated cellulose (ORC), collagen, gelatin, fibrin glue, chitosan or derivatives thereof.

The hemostatic material may be added on the surface of fibers or in the fibers. In order to provide the hemostatic material on the surface of fibers, the medical dressing is immersed or sprayed with the hemostatic material.

When the surface is treated by immersion, the hemostatic material such as oxidized cellulose (OC), oxidized regenerated cellulose (ORC), collagen, gelatin, fibrin glue, chitosan or derivatives thereof is added in one or more organic solvents, and then is coupled to the surface of fibers. Certainly, a coupling agent may be used. The treatment may further include drying.

The material for enhancing blood circulation may be the material emitting near infra-red and far infra-red. Preferably, the material for enhancing blood circulation may be metal or metal oxide. The metal may be titanium, germanium, or other material emitting near infra-red and far infra-red. The metal oxide may be zinc oxide, aluminum oxide, magnesium oxide or other material emitting near infra-red and far infra-red.

The material emitting near infra-red and far infra-red may be micro particles or nano particles mixed in the fiber material of the medical dressing of the present invention, wherein the particle size is smaller than 100 µm, and preferably smaller than 50 µm.

The material for absorbing odor is used for absorbing ammonia or other odor in a chemical or physical manner, wherein the material may be a metal oxide or active carbon material. The metal oxide may be zinc oxide, aluminum oxide, magnesium oxide or other material for absorbing odor. This material is added to the whole medical dressing or on the surfaces of the medical dressing.

The polymeric material which contains repeat units of urethane is used in the present invention. In comparison with the common biomedical material such as PTEE, silicon or PVC, thermoplastic polyurethane resin (TPU) or polymer including repeat units of urethane has soft segments and hard segments, so as to have elasticity and softness. Further, the polymer material including repeat units of urethane may be applicable to various processing procedures, and applicable in wound dressings due to great mechanical property, elasticity, adjustable hardness, blood compatibility and biocompatibility

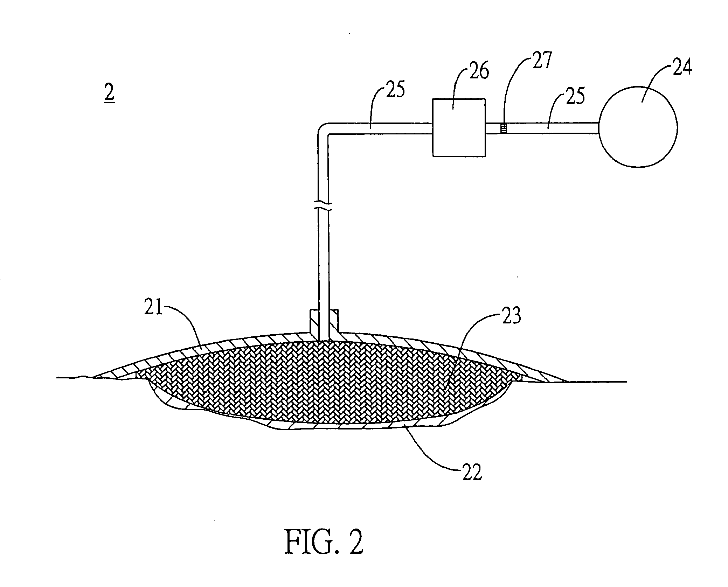

As shown in FIG. 2, a negative pressure wound therapy apparatus 2 includes a medical dressing 23; a sealing component 21 attached on the medical dressing 23 and identifying space between the seal component 21 and a tissue 22; a vacuum unit 24; and a communicating tube 25 having two end respectively connected to the sealing component 21 and the vacuum unit 24. The negative pressure wound therapy apparatus 2 is used for covering the wound via the medical dressing 23, so as to establish a local negative pressure environment. The vacuum unit 24 may be a pump, the communicating tube 25 may be a medical tube, and the sealing component 21 may be a semi-steel cup, which may be adequately deformed, or a soft film material.

The negative pressure therapy apparatus 2 further includes a container 26 disposed between the sealing component 21 and the vacuum unit 24, and connected to the communicating tube 25 for receiving wound fluid. Further, the negative pressure therapy apparatus 2 includes a filter 27 disposed in the communicating tube 25 between the container 26 and the vacuum unit 24.

The features and advantages of the present invention are illustrated, but not limited to, the following embodiments.

### Embodiments

### Synthesis of hydrophilic polyurethane material

### Preparation 1: synthesis of PVP-OH

In a reaction bottle, N-vinyl-2-pyrrolidone (VP) was added in and mixed with 179 mL of water-removed dimethylacetamide (DMAc), and then added with 2,2'-azobisisobutyronitrile (AIBN) and mercaptoethanol (ME) in sequence. This procedure was performed under nitrogen. The mixture was heated to 60°C, and the reaction was performed for 24 hours. After the reaction, DMAc was removed, the product was dissolved in 70 mL of dichloromethane and then precipitated in 700 mL of ethyl ether (1:10). The product was dried in a vacuum oven at 60°C for 24 hours. The product has the following structure PVP-OH. The amounts of N-vinyl-2-pyrrolidone, mercaptoethanol, and the product in each batch were shown in Table 1.

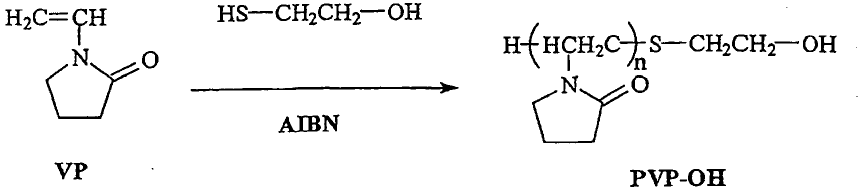

In the structure PVP-OH, n is an integer of from 1 to 440.

| batch | VP:ME | AIBN | Molecular weight | | |
|---|---|---|---|---|---|
| | | | Mn | Mw | PDI |
| #1 | 20:1 (0.60 mol/0.03 mol) | 1wt% (4.20 mmol) | 12,771 | 33,993 | 2.66 |
| #2 | 15:1 (0.60 mol/0.04 mol) | 1wt% (4.26 mmol) | 12,410 | 33,685 | 2.71 |
| #3 | 10:1 (0.60 mol/0.06 mol) | 1wt% (4.32 mmol) | 12,029 | 26,838 | 2.23 |
| #4 | 5:1 (0.30 mol/0.06 mol) | 1wt% (2.32 mmol) | 7,427 | 14,940 | 2.01 |
| #5 | 20:1 (0.40 mol/0.02 mol) | 21.0 mmol (5 times of #1) | 4,640 | 9,986 | 2.15 |
| #6 | 20:1 (0.40 mol/0.02 mol) | 42 mmol (10 times of #1) | 2,127 | 6200 | 2.92 |

### Preparation 2: synthesis of a PVP derivative having a diol group

PVP-OH (PVP-OH-980730-2, obtained from batch #3 in Preparation 1) was water-removed under vacuum for 12 hours. PVP-OH (46 g, 3.82 mmol) was added in 500 mL of a reaction bottle, which was connected to a stirring device and a condensation tube introduced with nitrogen, and 100 mL of water-removed dimethyl sulfoxide (DMSO) was added. The mixture was heated to 60°C for being completed dissolved. Then, 4,4'-diphenylmethane diisocyanate (MDI) (0.98g, 3.90mmol) was added, and the reaction was performed for 3 hours. The product, PVP-NCO, was obtained.

In the reaction bottle having PVP-NCO (46.98 g, 3.82 mmol), pentaerythritol (0.26 g, 1.86 mmol) was injected into the reaction bottle, and then the reaction was performed at 60°C for 4 hours. After the reaction, dibutyl amine (DBA) (0.05 g, 0.38 mmol) was added to remove the activity of NCO group. In the water bath at 60 to 70°C, DMSO was removed. Then, the residues were dissolved in dichloromethane, precipitated in ethyl ether (1:10) twice, and dried under vacuum at 60°C. The PVP derivative having a diol group was obtained.

The molecular weight of the PVP derivative having the diol group was analyzed by GPC, and the grafting ratio was analyzed by ¹H-NMR. The results showed that the PVP derivative had the molecular weight: Mn: 13,600; Mw: 28,500; and PDI = 2.1, and the grafting ratio was 108%. The PVP derivative has the structure of formula (III).

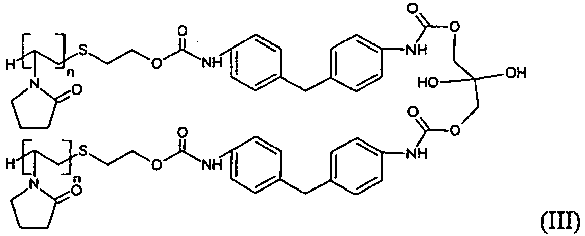

### Preparation 3: synthesis of a PEG derivative having a diol group

The commercial methyl ether polyethylene glycol (mPEG) (150 g, 0.03 mol) was added into a reaction bottle, and dewatered at 80°C for 2 hours. Then, 368 mL of dewatered DMSO was added, and the mixture was heated to 60°C for being completed dissolved. Subsequently, diphenyl-methane-diisocyanate (MDI) (7.66 g, 0.0306 mol) powder was added and dissolved in 30 mL of DMSO, and the reaction of the mixture was performed for 3 hours. The product was mPEG-NCO.

In the reaction bottle having mPEG-NCO solution (157.7 g, 0.03 mol), pentaerythritol (1.98 g, 0.015 mol) was injected into the reaction bottle. The reaction of the mixture was performed at 60°C for 4 hours. After the reaction, an adequate amount of DBA (0.39 g, 0.003 mol) was added to remove the activity of the NCO group. The DMSO was removed, and the residues were dissolved in dichloromethane and precipitated in ethyl ether (1:10) twice. The dried white solid was the PEG derivative having the diol group. The PEG derivative had the structure of formula (IV), and the molecular weight: Mn: 6,400; Mw: 8,300; and PDI: 1.3.

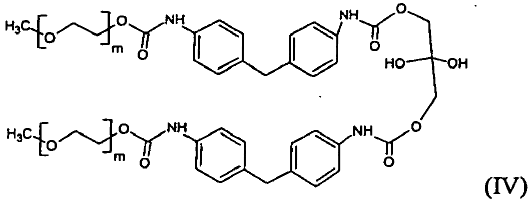

### Preparation 4: synthesis of polyurethane having side chains bound with hydrophilic segments

The polyurethane having side chains bound with hydrophilic segments was formed by solution polymerization, wherein 4,4'-diphenylmethane diisocyanate (MDI), PTMEG 1,000 and the chain elongation agent (PEG-diol or PVP-diol) and 1,4-BDO were used, and the solvent was N,N-dimethylethylamine. The reaction temperature was 65±5°C, and the reaction system included 20 wt% of solid. The compositions for the polymer synthesis were shown in Table 2 and Table 3.

**Table 2 Composition of polyurethane having PVP hydrophilic side chains**

| batch | composition | | | | | Molecular weight (Mw) |
|---|---|---|---|---|---|---|
| | PTMEG | 1-4 BDO | PVP₅₀₀₀-diol | MDI | PVP-diol content (wt%) | |
| #7 | 24.4g | 4.4g | 2.9g | 19.8g | 5.6% | 77219 |
| #8 | 20.8 g | 3.7g | 10g | 17.6g | 19.2% | 43949 |
| #9 | 16.0 g | 2.7g | 19.3g | 15.2g | 36.3% | 44749 |

**Table 3 Composition of polyurethane having PEG hydrophilic side chains**

| batch | composition | | | | | Molecular weight (Mw) |
|---|---|---|---|---|---|---|
| | PTMEG | 1-4 BDO | PEG_{5,000}-diol | MDI NCO=1.03 | PEG-diol content (wt%) | |
| #10 | 24.4 g | 4.4 g | 2.5g | 18.5g | 5.0% | 36629 |
| #11 | 23.2 g | 4.1 g | 4.7g | 21.1g | 8.9 % | 47520 |
| #12 | 21.2 g | 3.7g | 8.6g | 18.3g | 16.6% | 37669 |
| #13 | 16.9 g | 2.9g | 17.1g | 14.2g | 33.5% | 74389 |
| #14 | 12.6 g | 2.0g | 25.5g | 11.7g | 49.2% | 94325 |

### Test example 1: test of cell attachment

The *in vitro* cell attachment test was used for testing the cell attachment inhibition capability of the polyurethane having hydrophilic side chains. The conditions and method of the test was as follows. The cell strain was L-929, the cell population was 2x10⁴ cells/well (12-well culture plate), the culture condition was 7°C and 5% CO₂, the control group was 12-well culture plate, and the test sample was a circular film with a diameter of 21 mm.

The polyurethane having hydrophilic side chains and the polyurethane without hydrophilic side chains (control group) were formed as circular thin films (diameter: 20 mm, thickness: 0.5 mm), placed on the bottom of the 12-well culture plate, and the films were fixed with non-toxic rubber rings. 2 mL of cell suspension containing 2x10⁴ cells were added and cultured for 18 hours to make cells attached. Then, the medium was removed by pipette, and the unattached cells were removed by 2 mL of DPBS. 2 mL of neutral red was added for reaction for 1 hour. The cells were observed by microscope. Each well plate was divided into 5-6 areas to be taken photos for records. The number of attached cells was calculated per unit area, and then the average number of the attached cells in each well plate was calculated. Each film was tested for three times. The cell attachments on different films were compared. The inhibition of cell attachment was shown in Table 4 and Table 5.

**Table 4 Cell attachment on the polyurethane having PVP hydrophilic side chains**

| batch | PVP-diol content (wt%) | Cell number under 100X magnification |
|---|---|---|
| control | 0% | 139±18 |
| #7 | 5.6% | 82±16 |
| #8 | 19.2% | 28±11 |
| #9 | 36.3% | 2±2 |

**Table 5 Cell attachment on the polyurethane having PEG hydrophilic side chains**

| batch | PEG-diol content (wt%) | Cell number under 100X magnification |
|---|---|---|
| control | 0% | 139±18 |
| #10 | 5.0% | 62±12 |
| #11 | 8.9% | 44±9 |
| #12 | 16.6% | 14±6 |
| #13 | 33.5% | 1±1 |
| #14 | 49.2% | 0 |

### Test example 2: test of wound adhesion

Rats were used for testing wound adhesion inhibition of the polyurethane having hydrophilic side chains. The conditions and method of the test were as follows. A wound (2.5x2.5 cm) was made on the back of a rat. Three days after that, the test sample was fixed on the wound bed. On day 7, the rat was anesthetized, and the rat was tied with a stitch and fixed at a corner. The test sample was torn from the wound bed at 45° in the opposite direction, and the pulling force that the test sample applied on the wound bed was measured by a force gauge meter. The rate of tearing the test sample was 1 cm/s. The results were shown in Table 6.

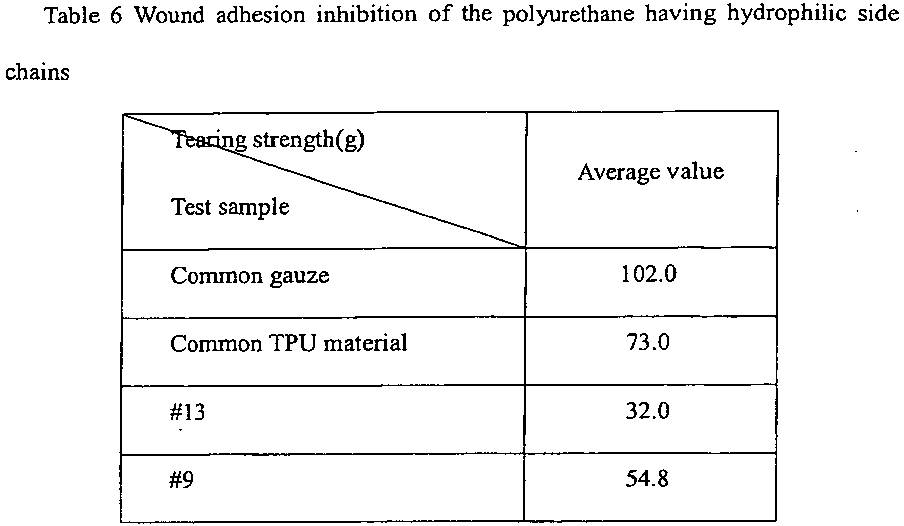

### Preparation 5: Formation of thermoplastic polyurethane fibers

The following polyurethane materials were used:
(1) Tecothane® (Lubrizol Corporation);
(2) ISOTHANE 1000 (Great Eastern Resins Industrial Co. Ltd.);
(3) ISOTHANE 3000 (Great Eastern Resins Industrial Co. Ltd).

The fibers were formed by melt spinning (mold number of spinning test machine: JOYSPPL03, Yi-Shiang Engineering Ltd.) The polyurethane needed to have moisture content less than 100 ppm before melt spinning. The conditions for melt spinning and the property of fibers were shown in Table 7.

**Table 7 Processing conditions and property of polyurethane materials**

| No. | Material specification | hardness | Spinneret pore number | Spinning temperature (°C) | Spinning rate (m/min) | Spinning amount (g/min) | Fineness (den/f) | Strength (g/d) | Elongation (%) |
|---|---|---|---|---|---|---|---|---|---|
| #15 | Lubrizol TT-1085A (polyether) | 85A | 24 | 225 | 2000 | 25 | 100/24 | 1.01 | 169.3 |
| #16 | Lubrizol TT-1095A (polyether) | 95A | 24 | 225 | 2000 | 21.5 | 74/24 | 2.21 | 134.1 |
| #17 | Lubrizol TT-1055D (polyether) | 55D | 24 | 230 | 2500 | 23 | 82.6/24 | 2.44 | 113.8 |
| #18 | Lubrizol TT-1065D (polyether) | 65D | 24 | 240 | 2500 | 28 | 99/24 | 2.14 | 131 |
| #19 | GRECO 1055D (polyether) | 55D | 24 | 230 | 1500 | 19 | 106.5/24 | 2.42 | 153.7 |
| #20 | Lubrizol TT-1055D (polyether) | 55D | 24 | 230 | 1500 | 20.3 | 125/24 | 1.80 | 177 |
| #21 | GRECO 1065D (polyester) | 65D | 24 | 240 | 2500 | 27.2 | 98.4/24 | 2.24 | 103.7 |
| #22 | GRECO 3065D (polyether) | 65D | 24 | 240 | 2000 | 23.0 | 106/24 | 1..83 | 120.3 |
| #23 | polyether | 65D | 24 | 235 | 1500 | 20.3 | 125/24 | 1.77 | 208.6 |
| #24 | polyether | 65D | 24 | 240 | 500 | 18 | 1000/72 | 1.19 | 351.4 |

### Preparation 6: Formation of hydrophilic polyurethane fibers

The TPU aliphatic hydrophilic polyurethane Tecophilic® (Lubrizol Corporation) and the TPU aromatic hydrophilic polyurethane BE-5038A (Coating Chemical Industry Co. Ltd.) were used.

The fibers were formed by melt spinning (mold number of spinning machine KLB-100, Killion Extruders Inc.; mold number of spinning test machine: JOYSPPL03, Yi-Shiang Engineering Ltd.) The polyurethane needed to have moisture content less than 100 ppm before melt spinning. The conditions for melt spinning and the property of fibers were shown in Table 8.

**Table 8 Processing conditions and property of hydrophilic polyurethane materials**

| Material specification | hardness | Spinneret pore number | Spinning temperature (°C) | Spinning rate (m/min) | Spinning amount (g/min) | Fineness (den/f) | Strength (g/d) | Elongation (%) |
|---|---|---|---|---|---|---|---|---|
| #25(BE-5038A) | 88A | 12 | 190 | 300 | 6.2 | 199.9/12 | 0.59 | 144.7 |
| #26(Tecophilic-60D60) | 41D | 12 | 190 | 500 | 6.8 | 144.2/12 | 0.49 | 241.3 |
| #27(Tecophilic-SP-93A-100) | 83A | 12 | 180 | 300 | 4.3 | 130.6/12 | 0.19 | 300.9 |
| #28(BE-5038A) | 88A | 24 | 190 | 1500 | 21.0 | 79.87/24f | 1.43 | 124.0 |

### Preparation 7: Formation of medical dressing

The polyurethane fibers formed from Preparation 5 were woven into a circular knitting structure, so as to form a medical dressing of the present invention. The circular knitting machine is the 34KL knitting machine from Kuan Yu Machinery Co., Ltd. The conditions for the circular knitting and property test were shown in Table 9.

**Table 9 Conditions for circular knitting and property test**

| No. | Fibers | Hardness | Linear support portions | Knitting number | Speed | Thickness |
|---|---|---|---|---|---|---|
| S1 | Preparation 5(#21) | 65D | polyester (PET) | 19 | 10rpm/min | 5 mm |
| S2 | Preparation 5(#19) | 55D | polyester (PET) | 28 | 10rpm/min | 5 mm |

### Test example 3: Test of removal rate of would fluid

A silicon cavity (5x5x4 cm) mimicked wounds where pressure sensors were respectively buried in the center and lateral sides of the silicon cavity was prepared. The blood mimicking fluid was introduced to two sides of the bottom of the silicon cavity via a 22G steel needle. The cottons and fiber dressings were tested for the fluid removal rate. The blood mimicking fluid was Lot#CGB3120, Model 046, purchased from Computerized Imaging Reference Systems, Inc. The test pressure was 635 mm-Hg, and the test results were shown in Table 10.

**Table 10 Property test of the medical dressing of the present invention and the conventional dressings**

| Test sample No. | Processing for dressing | Thickness (mm) | Overall density (g/cm³) | Area of surface opening (mm²) | | Pressure difference (mm/Hg) | Flow rate of blood mimicking fluid (mg/sec) |
|---|---|---|---|---|---|---|---|
| | | | | upper | lower | | |
| Commercial PU foams | foaming | 30.0 | 0.021 | 0.283 | 0.283 | 1.0 | 98.04 |
| TPU non-woven cloth | Thermal spray | 2.26 | 0.077 | 0.004 | 0.004 | 4.0 | 27.7 |
| Polyester steric woven material | Short fiber needle punching | 8.75 | 0.005 | 0.442 | 0.448 | 2.0 | 110.5 |
| Fiber dressing (S1) | Circular knitting | 2.94 | 0.083 | 0.096 | 0.108 | 3.0 | 111.39 |

The invention has been described using exemplary preferred embodiments. However, it is to be understood that the scope of the invention is not limited to the disclosed arrangements. The scope of the claims, therefore, should be accorded the broadest interpretation, so as to encompass all such modifications and similar arrangements.

## Claims

1. A medical dressing, comprising:
a first and a second woven layers respectively comprising a fiber of polyurethane or a yarn of polyurethane; and
a plurality of linear support portions woven between the first and second woven layers,
wherein the first and the second woven layer respectively have a surface with a plurality of openings, with the average area of the openings being in a range from 1×10⁻⁴ to 1×10² mm²,
wherein the medical dressing has an overall density in a range from 0.01 to 0.99 g/cm³, and
wherein the fiber or yarn has a hardness in a range from Shore 50A to Shore 80D.

2. The medical dressing of claim 1, wherein the fiber or the yarn is in a range from 0.01 denier to 1000 denier.

3. The medical dressing of claim 1, wherein the first and the second woven layers have a weaving structure, a knitting structure, a circular knitting structure or a warp knitting structure.

4. The medical dressing of claim 1, wherein the polyurethane comprises aliphatic polyurethane, aromatic polyurethane, aliphatic polyurethane having a hydrophilic segment, aromatic polyurethane having a hydrophilic segment or a combination thereof.

5. The medical dressing of claim 1, wherein the polyurethane is the aliphatic polyurethane having a hydrophilic segment or the aromatic polyurethane having a hydrophilic segment; and the hydrophilic segment is disposed on a main chain or is a side chain bound to the aliphatic polyurethane or the aromatic polyurethane.

6. The medical dressing of claim 5, wherein the hydrophilic segment is bound to the aliphatic polyurethane or the aromatic polyurethane via an ester bond or a urethane bond.

7. The medical dressing of claim 6, wherein the hydrophilic segment comprises polyethylene glycol, polyethylene oxide, polyvinylpyrrolidone, polyvinyl alcohol, polyacrylic acid, polymethacrylic acid or a combination thereof.

8. The medical dressing of claim 5, wherein the hydrophilic segment has a molecular weight in a range from 100 to 500,000.

9. The medical dressing of claim 1, further comprising at least an antimicrobial component selected from the group consisting of silver, iodine, zinc, copper and an antibiotic.

10. The medical dressing of claim 9, wherein the antimicrobial component is coupled to the surface of the first woven layer, the second woven layer or the plurality of linear support portions via a coupling agent.

11. The medical dressing of claim 10, wherein the coupling agent is one or more of polyurethane and amphiphilic polymer.

12. A negative pressure wound therapy apparatus, comprising:
a medical dressing of any of claims 1 to 11;
a sealing component attached on the medical dressing;
a vacuum unit; and
a communicating pipe having two ends respectively connected to the sealing component and the vacuum unit.

## Patentansprüche

1. Medizinischer Verband umfassend:
eine erste sowie eine zweite Gewebeschicht, welche jeweils eine Polyurethanfaser oder ein Polyurethangarn enthalten, sowie
eine Vielzahl von linearen Trägergeweben zwischen der ersten und der zweiten Gewebeschicht,
wobei die erste und die zweite Gewebeschicht jeweils eine Oberfläche mit einer Vielzahl von Öffnungen aufweist, wobei die Durchschnittsfläche der Öffnungen in einem Bereich zwischen 1x10⁻⁴ und 1x10² mm² liegt,
wobei der medizinische Verband eine Gesamtdichte in einem Bereich zwischen 0,01 und 0,99 g/cm³ aufweist, und
wobei die Faser oder das Garn eine Härte in einem Bereich von Shore 50A bis Shore 80D aufweist.

2. Medizinischer Verband nach Anspruch 1, wobei die Faser oder das Garn in einem Bereich zwischen 0,01 Denier und 1.000 Denier liegt.

3. Medizinischer Verband nach Anspruch 1, wobei die ersten und zweiten Gewebeschichten eine Gewebestruktur, eine Gestrickstruktur, eine zirkuläre Gestrickstruktur oder eine Kettenwirkstruktur aufweisen.

4. Medizinischer Verband nach Anspruch 1, wobei das Polyurethan aliphatisches Polyurethan, aromatisches Polyurethan, aliphatisches Polyurethan mit einem hydrophilen Segment, aromatisches Polyurethan mit einem hydrophilen Segment oder einer Mischung hiervon umfasst.

5. Medizinischer Verband nach Anspruch 1, wobei das Polyurethan ein aliphatisches Polyurethan mit einem hydrophilen Segment oder ein aromatisches Polyurethan mit einem hydrophilen Segment ist, und, wobei das hydrophile Segment an der Hauptkette angeordnet ist oder eine Seitenkette ist, die an das aliphatische Polyurethan oder das aromatische Polyurethan gebunden ist.

6. Medizinischer Verband nach Anspruch 5, wobei das hydrophile Segment an das aliphatische Polyurethan oder das aromatische Polyurethan über eine Esterbindung oder über eine Urethanbindung gebunden ist.

7. Medizinischer Verband nach Anspruch 6, wobei das hydrophile Segment Polyethylenglycol, Polyethylenoxid, Polyvinylpyrrolidon, Polyvinylalkohol, Polyacrylsäure, Polymethacrylsäure oder eine Mischung hiervor umfasst.

8. Medizinischer Verband nach Anspruch 5, wobei das hydrophile Segment ein Molekulargewicht in einem Bereich zwischen 100 und 500.000 aufweist.

9. Medizinischer Verband nach Anspruch 1, welcher des Weiteren wenigstens eine antimikrobielle Komponente ausgewählt aus der Gruppe besteht aus Silber, lod, Zink, Kupfer und einem Antibiotikum umfasst.

10. Medizinischer Verband nach Anspruch 9, wobei die antimikrobielle Komponente an die Oberfläche der ersten Gewebeschicht, der zweiten Gewebeschicht oder die Vielzahl von linearen Trägerteilen über ein Kupplungsmittel gekoppelt ist.

11. Medizinischer Verband nach Anspruch 10, wobei das Kupplungsmittel eines oder mehrere von Polyurethan oder amphiphilen Polymer ist.

12. Unterdruck-Wundtherapievorrichtung umfassend:
einen medizinischen Verband nach einem der Ansprüche 1 bis 11,
eine Dichtkomponente, welche an den medizinischen Verband angebracht ist,
eine Vakuumeinheit und
ein verbindendes Rohr mit zwei Enden, welche jeweils mit der Dichtkomponente und der Vakuumeinheit verbunden sind.

## Revendications

1. Pansement médical comprenant :
une première et une seconde couche tissée comprenant respectivement une fibre de polyuréthanne ou un fil de polyuréthanne ; et
une pluralité de parties de support linéaires tissées entre les première et seconde couches tissées,
dans,lequel la première et la seconde couche tissée ont respectivement une surface avec une pluralité d'ouvertures, dont la surface moyenne des ouvertures est de l'ordre de 1 x 10⁻⁴ à 1 x 10² mm²,
dans lequel le pansement médical a une densité globale de l'ordre de 0,01 à 0,99 g/cm³, et
dans lequel la fibre ou le fil a une dureté de l'ordre de 50 Shore A à 80 Shore D.

2. Pansement médical selon la revendication 1, dans lequel la fibre ou le fil est dans une plage de 0,01 denier à 1000 deniers.

3. Pansement médical selon la revendication 1, dans lequel la première et la seconde couche tissée ont une structure de tissage, une structure de tricot, une structure de tricot circulaire ou une structure à mailles jetées.

4. Pansement médical selon la revendication 1, dans lequel le polyuréthanne comprend le polyuréthanne aliphatique, le polyuréthanne aromatique, le polyuréthanne aliphatique ayant un segment hydrophile, le polyuréthanne aromatique ayant un segment hydrophile ou leur combinaison.

5. Pansement médical selon la revendication 1, dans lequel le polyuréthanne est le polyuréthanne aliphatique ayant un segment hydrophile ou le polyuréthanne aromatique ayant un segment hydrophile ; et le segment hydrophile est disposé sur une chaîne principale ou est une chaîne latérale reliée au polyuréthanne aliphatique ou au polyuréthanne aromatique.

6. Pansement médical selon la revendication 5, dans lequel le segment hydrophile est relié au polyuréthanne aliphatique ou au polyuréthanne aromatique via une liaison d'ester ou une liaison d'uréthanne.

7. Pansement médical selon la revendication 6, dans lequel le segment hydrophile comprend le polyéthylèneglycol, l'oxyde de polyéthylène, la polyvinylpyrrolidone, l'alcool polyvinylique, l'acide polyacrylique, l'acide polyméthacrylique ou une de leurs combinaisons.

8. Pansement médical selon la revendication 5, dans lequel le segment hydrophile a un poids moléculaire de l'ordre de 100 à 500000.

9. Pansement médical selon la revendication 1, comprenant en outre au moins un composant antimicrobien choisi dans le groupe comprenant l'argent, l'iode, le zinc, le cuivre et un antibiotique.

10. Pansement médical selon la revendication 9, dans lequel le composant antimicrobien est couplé à la surface de la première couche tissée, de la seconde couche tissée ou à la pluralité de parties de support linéaires via un agent de couplage.

11. Pansement médical selon la revendication 10, dans lequel l'agent de couplage est l'un ou plusieurs parmi le polyuréthanne et un polymère amphiphile.

12. Appareil de thérapie des blessures par pression négative, comprenant :
un pansement médical selon l'une quelconque des revendications 1 à 11 ;
un composant d'étanchéité fixé sur le pansement médical ;
une unité de vide ; et
un tuyau de communication ayant deux extrémités respectivement raccordées au composant d'étanchéité et à l'unité de vide.
